# EUROPEAN PATENT APPLICATION

(11) **EP 4 803 532 A1**
(43) Date of publication of application: **09.09.2026**
(21) Application number: 24885036.4
(22) Date of filing: 04.11.2024
(51) Int. Cl.: C07K 14/01, A61K 39/12, A61K 9/51, A61P 31/20, C07K 19/00, G01N 33/569, C12N 15/34, C12N 15/62

(54) **IMMUNOGENIC COMPOSITION OF AFRICAN SWINE FEVER VIRUS CD2V PROTEIN AND USE THEREOF**

(30) Priority: 03.11.2023 CN 202311466483
(71) Applicant: Beijing Zhongke Lanyu Biotechnology Co., Ltd., Beijing 100096 (CN); Institute of Biophysics of the Chinese Academy of Sciences, Beijing 100101 (CN)
(72) Inventor: GAO, Guangxia, Beijing 100101 (CN); HUANG, Linlong, Beijing 100101 (CN); ZHANG, Xiaolin, Beijing 100101 (CN); ZHANG, Jialong, Beijing 102206 (CN); ZHANG, Yunxing, Beijing 102206 (CN); NIE, Xiaohua, Beijing 100101 (CN); FAN, Xiuli, Beijing 102206 (CN); ZHANG, Liguo, Beijing 100101 (CN); WANG, Xiangxi, Beijing 100101 (CN); GAO, Pu, Beijing 100101 (CN)
(74) Representative: Delbarba, Andrea
(86) International application number: PCT/CN2024/129778
(87) International publication number: WO 2025/093041

(57) **Abstract**

An immunogenic fragment of African swine fever virus CD2V protein, a recombinant protein, an immunogenic composition, and a use thereof. The provided immunogenic fragment of African swine fever virus CD2V protein or a variant thereof with immunogenicity can greatly improve the expression quantity while retaining the strong immunocompetence.

## Description

### TECHNICAL FIELD

The present disclosure relates to an immunogenic composition, and in particular to the African Swine Fever Virus CD2V protein or an immunogenic fragment thereof, the corresponding encoding nucleotide sequence, immunogenic composition and use thereof.

### BACKGROUND

African swine fever is a viral disease caused by infection with African swine fever virus (ASFV). The acute form clinically presents with high fever, depression, anorexia, skin cyanosis, and hemorrhage in various organs. The disease exhibits high infectivity and lethality, with morbidity and mortality rates reaching 100%.

Due to the large genomic structure and complex immune escape mechanisms of ASFV, the development of effective vaccines is very difficult, and so far, there are no safe and effective vaccines for epidemic prevention and control. Previous studies on African swine fever vaccines have shown that "inactivated vaccines" can induce high levels of humoral immune responses but cannot provide immune protection. Therefore, the current design methods for African swine fever vaccines mainly focus on attenuated vaccines and subunit vaccines.

Attenuated vaccines have made faster research progress in various countries, but their existing problems have become increasingly exposed. The protection provided by attenuated vaccines is usually only against homologous strains of the same genotype and cannot resist heterologous virus attacks. Attenuated vaccines usually also have related adverse side effects, such as skin damage and joint swelling. In addition, attenuated vaccines may lead to chronic or persistent infections and may regain virulence.

Compared with the attenuated vaccines, the subunit vaccines provide a targeted approach with fewer side effects and higher safety. Moreover, previous research results have shown that a variety of African swine fever virus antigens can induce neutralizing antibodies and provide partial immune protection, which provides the possibility for developing safe and effective African swine fever vaccines. With the in-depth research on the structural biology and immunology of African swine fever virus, the design of effective subunit vaccines that can produce protective antibodies and specific cellular immune responses has become one of the hotspots in the field.

### SUMMARY

To solve one of the above-mentioned technical problems in the prior art, the present disclosure provides an immunogenic composition comprising two different domains of the CD2v protein of African swine fever virus (ASFV), and use thereof.

According to one aspect of the present disclosure, there is provided an immunogenic fragment or an immunogenic variant thereof, wherein the immunogenic fragment comprises at least the amino acid fragment at positions 18 to 100 of the amino acid sequence shown in SEQ ID NO: 1, and/or comprises at least the amino acid fragment at positions 117 to 200 of the amino acid sequence shown in SEQ ID NO: 1.

In some embodiments, the immunogenic fragment may comprise at least the amino acid fragment at positions 18 to 100 of the amino acid sequence shown in SEQ ID NO: 1. In some embodiments, the immunogenic fragment of the CD2v protein may comprise at least the amino acid fragment at positions 18 to 100, 19 to 100, 18 to 101, 19 to 101, 18 to 102, 19 to 102, 18 to 103, 19 to 103, 18 to 104, 19 to 104, 18 to 105, 19 to 105, 18 to 106, 19 to 106, 18 to 107, 19 to 107, 18 to 108, 19 to 108, 18 to 109, or 19 to 109 of the amino acid sequence shown in SEQ ID NO: 1. In preferred embodiments, the immunogenic fragment may comprise the amino acid fragment at positions 18 to 100, 18 to 101, 18 to 102, 18 to 103, 18 to 104, 18 to 105, 18 to 106, 18 to 107, 18 to 108, or 18 to 109 of the amino acid sequence shown in SEQ ID NO: 1.

In some embodiments, the immunogenic fragment comprises at least the amino acid fragment at positions 18 to 106 of the amino acid sequence shown in SEQ ID NO: 1.

In some embodiments, the immunogenic fragment comprises at most the amino acid fragment at positions 16 to 110 of the amino acid sequence shown in SEQ ID NO: 1. In some embodiments, the immunogenic fragment comprises at most the amino acid fragment at positions 18 to 110 of the amino acid sequence shown in SEQ ID NO: 1.

In some embodiments, the immunogenic fragment may have the amino acid sequence shown in SEQ ID NO: 5, SEQ ID NO: 6, SEQ ID NO: 7, and/or SEQ ID NO: 8, or an amino acid sequence having at least 85% sequence identity thereto.

In some embodiments, the immunogenic fragment may comprise at least the amino acid fragment at positions 117 to 200 of the amino acid sequence shown in SEQ ID NO: 1. In some embodiments, the immunogenic fragment comprises at least the amino acid fragment at positions 114 to 204 of the amino acid sequence shown in SEQ ID NO: 1.

In some embodiments, the immunogenic fragment may comprise at least the amino acid fragment at positions 108 to 204, 109 to 204, 110 to 204, 111 to 204, 112 to 204, 113 to 204, 114 to 204, 115 to 204, 116 to 204, or 117 to 204 of the amino acid sequence shown in SEQ ID NO: 1. In some embodiments, the immunogenic fragment may have the amino acid sequence shown in SEQ ID NO: 9, SEQ ID NO: 10, or SEQ ID NO: 11, or an amino acid sequence having at least 85% sequence identity thereto.

In some embodiments, the immunogenic variant of the immunogenic fragment has an amino acid sequence that is at least 85%, at least 90%, at least 95%, at least 96%, at least 97%, at least 98%, at least 99%, or at least 100% identical to the immunogenic fragment.

According to another aspect of the present disclosure, there is provided a recombinant protein, the recombinant protein comprising: a first domain comprising the above-mentioned immunogenic fragment or the immunogenic variant thereof; and a second domain comprising a scaffold polypeptide for forming nanoparticles.

In some embodiments, the second domain, as a scaffold polypeptide, is capable of self-assembling or pairing with another scaffold polypeptide to assemble into nanoparticles, while displaying the immunogenic fragment or its variant of the first domain on the surface of the nanoparticles.

In some embodiments, the second domain may be selected from, for example, I53-34A, I53-34B, I53-40A, I53-40B, I53-47A, I53-47B, I53-50A, I53-50B, I53-51A, I53-51B, I52-03A, I52-03B, I52-32A, I52-32B, I52-33A, I52-33B, I32-06A, I32-06B, I32-19A, I32-19B, I32-28A, I32-28B, I53-40A.1, I53-40B.1, I53-47A.1, I53-47A.1 NegT2, I53-47B.1, I53-47B.1 NegT2, I53-50A.1, I53-50A.1 NegT2, I53-50A.1 PostT1, I53-50B4 PostT1, LS (Lumazine synthase), E2P (dihydrolipoamide acetyltransferase) and I3.

In specific embodiments, the second domain may be selected from I52-32A, I52-32B, I53-50A, I53-50B, I32-28A, I32-28B, E2P, and I3.

In some embodiments, the first domain and the second domain together form a fusion protein. In some embodiments, the first domain is directly linked to the second domain. In some embodiments, the first domain is linked to the second domain via a linker.

According to another aspect of the present disclosure, there is provided a nucleic acid molecule encoding the above-mentioned immunogenic fragment or the immunogenic variant thereof or the above-mentioned recombinant protein of the present disclosure.

According to yet another aspect of the present disclosure, there is provided an expression vector comprising the above-mentioned nucleic acid molecule of the present disclosure.

In some embodiments, the expression vector may be selected from viral or bacterial vectors, such as, but not limited to, African swine fever virus vectors, lentiviral vectors, avian poxvirus vectors, canine distemper virus vectors, herpesvirus vectors, varicella virus vectors, adenoviral vectors, adeno-associated viral vectors, and the like.

According to yet another aspect of the present disclosure, there is provided a host cell comprising the above-mentioned nucleic acid molecule of the present disclosure, or capable of expressing the above-mentioned immunogenic fragment or the immunogenic variant thereof or the above-mentioned recombinant protein of the present disclosure.

In some embodiments, the host cell is a prokaryotic cell or a eukaryotic cell.

In some embodiments, the prokaryotic cell may be selected from *Escherichia coli* or *Bacillus subtilis*, etc., such as *Escherichia coli* BL21, T7E, C41, Arctic, etc.

In some embodiments, the eukaryotic cell may be selected from yeast cell, insect cell, plant cell, animal cell, etc., such as yeast cell, CHO cell, 293 cell, Vero cell, or NSO cell, etc.

According to yet another aspect of the present disclosure, there is provided a nanoparticle comprising the above-mentioned immunogenic fragment or the immunogenic variant thereof or the above-mentioned recombinant protein of the present disclosure. The immunogenic fragment or the variant thereof is displayed on the surface of the nanoparticle.

In some embodiments, the nanoparticle comprises a 60-mer formed by self-assembly of scaffold proteins, which may be selected from, for example, LS, E2P, and I3.

In some embodiments, the nanoparticle comprises a 60-mer formed by paired assembly of the scaffold protein in the recombinant protein with another scaffold protein, such as I53-34A paired with I53-34B, I53-40A paired with I53-40B, I53-47A paired with I53-47B, I53-50A paired with I53-50B, I53-51A paired with I53-51B, I52-03A paired with I52-03B, I52-32A paired with I52-32B, I52-33A paired with I52-33B, I32-06A paired with I32-06B, I32-19A paired with I32-19B, and I32-28A paired with I32-28B.

In alternative embodiments, the nanoparticles may comprise one or more immunogenic fragments or immunogenic variants thereof of the present disclosure. In exemplary embodiments, the nanoparticles may comprise two immunogenic fragments or immunogenic variants thereof of the present disclosure, i.e., comprising at least the amino acid fragment at positions 18 to 100 of the amino acid sequence shown in SEQ ID NO: 1 and comprising at least the amino acid fragment at positions 117 to 200 of the amino acid sequence shown in SEQ ID NO: 1.

According to yet another aspect of the present disclosure, there is provided an immunogenic composition comprising: the above-mentioned immunogenic fragment or the immunogenic variant thereof, or the above-mentioned recombinant protein, the above-mentioned nucleic acid molecule, the above-mentioned host cell or the above-mentioned nanoparticle of the present disclosure; and a pharmaceutically acceptable carrier.

In some embodiments, the immunogenic composition may comprise: one or more of the above-mentioned immunogenic fragments or immunogenic variants thereof, or the above-mentioned recombinant proteins, or the above-mentioned nanoparticles of the present disclosure, or the nucleic acid molecules encoding one or more of the above-mentioned immunogenic fragments or immunogenic variants thereof or above-mentioned recombinant proteins, or expression vectors of the present disclosure.

In some embodiments, the immunogenic composition may further comprise an additional African swine fever virus antigen.

In some embodiments, the immunogenic composition may further comprise a pharmaceutically acceptable carrier.

In some embodiments, the pharmaceutically acceptable carrier includes an adjuvant, and the adjuvant includes: polymers of acrylic acid or methacrylic acid, maleic anhydride and alkenyl derivative polymers; immunostimulatory sequences (ISS), such as oligodeoxynucleotide sequences with one or more unmethylated CpG units (CpG ODN); water-in-oil (W/O) adjuvants, oil-in-water (O/W) adjuvants or oil-in-water-in-oil (W/O/W) adjuvants, such as Freund's adjuvant, SPT emulsion, MF59, ISA 206, ISA72, Adjuvant-65, SAF, etc.; cationic lipids containing quaternary ammonium salts such as DDA; cytokines; aluminum hydroxide or aluminum phosphate; saponins (e.g., Quil A, QS-21, GPI-0100); or any combination or mixture thereof.

In a preferred embodiment, the saponin is Quil A, QS-21, or GPI-0100.

In a preferred embodiment, the adjuvant includes an emulsion; the emulsion is an SPT emulsion, an MF59 emulsion, or the emulsion is formed by a combination of an oil and an emulsifier, and the emulsion may be based on light liquid paraffin oil, isoprenoid oils produced by oligomerization of olefins (such as squalane or squalene oil, oils produced by oligomerization of olefins, particularly isobutylene or decene), esters of linear alkyl-containing acids or alcohols (more particularly vegetable oils, ethyl oleate, propylene glycol di(caprylate/caprate), glycerol tri(caprylate/caprate) or propylene glycol dioleate), esters of branched-chain fatty acids or alcohols (particularly isostearates); the emulsifier is a nonionic surfactant (particularly esters of polyoxyethylenated fatty acids (e.g., oleic acid), esters of sorbitan, esters of mannitol dianhydride (such as anhydrous mannitol monooleate), esters of aliphatic diols, esters of glycerol, esters of polyglycerol, esters of propylene glycol and esters of oleic acid, esters of isostearic acid, esters of ricinoleic acid, or esters of hydroxystearic acid, which may be ethoxylated, ethers of fatty alcohols and polyols (e.g., oleyl alcohol), and polyoxypropylene-polyoxyethylene block copolymers (particularly L121)).

In a preferred embodiment, the polymer of acrylic acid or methacrylic acid is a cross-linked polymer of acrylic acid or methacrylic acid, in particular the compound carbomer, which is cross-linked with a polyalkenyl ether of a sugar or a polyol, preferably Carbopol 974P, 934P and 971P.

In a preferred embodiment, the copolymer of cis-butenedioic anhydride (maleic anhydride) and alkenyl derivatives is EMA which is a copolymer of cis-butenedioic anhydride and ethylene. In a preferred embodiment, the adjuvant is Gel 01 adjuvant.

In some embodiments, the immunogenic composition may be administered orally, intradermally, intramuscularly or intranasally.

According to yet another aspect of the present disclosure, there is provided use of one or more of the above-mentioned immunogenic fragments or immunogenic variants thereof or the above-mentioned recombinant proteins, the nucleic acid molecules encoding one or more of the recombinant proteins, expression vectors, the above-mentioned host cells, the above-mentioned nanoparticles or the above-mentioned immunogenic compositions of the present disclosure in the preparation of a medicament for preventing and/or treating African swine fever virus infection in a subject.

According to yet another aspect of the present disclosure, there is provided a method for preventing and/or treating African swine fever virus infection in a subject.

In some embodiments, the subject refers to a mammal. In some embodiments, the subject is a Suidae animal, such as a pig. In some embodiments, the individual or subject may be a wild boar (*Sus scrofa*), domestic pig (*Sus scrofa domesticus*), warthog (*Potamochoerus*), forest hog (*Hylochoerus*), giant forest hog (*Hylochoerus*), African wild boar (*Potamochoerus*) and feral pig.

In some embodiments, the African swine fever virus infection may be a pathogenic African swine fever virus infection. In some embodiments, the symptoms or diseases of pathogenic African swine fever virus infection may be selected from the group consisting of: African swine fever, acute African swine fever, chronic African swine fever, death from illness, death, sudden death, fever, high fever, anorexia, lethargy, weakness, lack of appetite, recumbency, erythema, cyanotic skin maculopathy, dysentery, constipation, abdominal pain, respiratory symptoms, cough, vomiting, dyspnea, nasal discharge and conjunctival secretions, hemorrhage, epistaxis, abortion, leukopenia, and thrombocytopenia.

According to yet another aspect of the present disclosure, there is provided a kit for detecting African swine fever virus infection, the kit comprising the above-mentioned immunogenic fragment or the immunogenic variant thereof or the above-mentioned recombinant protein of the present disclosure.

According to yet another aspect of the present disclosure, there is provided use of the above-mentioned immunogenic fragment or the immunogenic variant thereof or the above-mentioned recombinant protein of the present disclosure in the preparation of a kit for detecting African swine fever virus infection.

In some embodiments, the sample is selected from a body fluid or tissue sample in a subject. In some embodiments, the sample may be selected from a blood, saliva or serum sample.

### BRIEF DESCRIPTION OF DRAWINGS

Figure 1 exemplarily shows an electron microscopy image of the assembly effect after fusing the 18-100 truncated fragment of the extracellular domain 1 of the African swine fever virus CD2v protein with the I3 protein.
Figure 2 exemplarily shows an electron microscopy image of the assembly effect after fusing the 114-204 truncated fragment of the extracellular domain 2 of the African swine fever virus CD2v protein with the I53-50A protein and assembling with I53-50B.
Figure 3 shows the binding strength results of different recombinant proteins of the extracellular domain 1 of the African swine fever virus CD2v protein with African swine fever positive serum.
Figure 4 shows the binding strength results of different recombinant proteins of the extracellular domain 2 of the African swine fever virus CD2v protein with African swine fever positive serum.
Figure 5 shows the serum antibody levels in mice immunized with different recombinant proteins of the extracellular domain 1 of the African swine fever virus CD2v protein.
Figure 6 shows the serum antibody levels in mice immunized with different recombinant proteins of the extracellular domain 2 of the African swine fever virus CD2v protein.

### DETAILED DESCRIPTION OF THE INVENTION

In order to make the objectives, technical solutions and advantages of the present disclosure clearer and more understandable, the present disclosure is described in further detail below in connection with examples. The specific examples described herein are for the sole purpose of explaining the present disclosure and are not intended to constitute any limitation of the present disclosure. In addition, in the following description, descriptions of publicly known structures and techniques are omitted to avoid unnecessary confusion about the concepts of the present disclosure. Such structures and techniques are also described in many publications.

The genome of African swine fever virus (ASFV) exceeds 170 kb and contains more than 150 open reading frames (ORFs). The virus particle has a diameter exceeding 200 nm. The virus forms viral factories around the nucleus, where viral replication and assembly occur. The ASFV particle is icosahedral and has a multi-layered envelope structure, namely an internal core (also called a nucleoid or viral nucleoid), a core shell, an inner envelope, a capsid, and an external envelope (composed mainly of lipids and a small amount of proteins). Proteins encoded by ASFV play important roles in virus assembly, DNA replication and repair, and gene expression. In addition, the ASFV genome encodes many proteins related to immune escape, including proteins that inhibit type I interferon and induce apoptosis, such as DP96R, MGF-505-7R and pE199L proteins.

The envelope protein CD2v is encoded by the EP402R gene, also known as the pEP402R protein, and contains 360 amino acids. It is named CD2v because the amino acid sequence encoding the immunoglobulin-like domain in the extracellular region of the protein is highly similar to that of the host cell CD2. The CD2v protein is a glycoprotein assembled from one signal peptide, two extracellular immunoglobulin-like domains, a transmembrane region, and an intracellular domain, wherein the intracellular domain comprises one acidic domain and one proline-rich repeat sequence. CD2v is a glycoprotein and facilitates the binding of ASFV-infected cells to red blood cells in porcine blood, thereby promoting viral dissemination within the host. CD2v plays an important role in viral replication, spread, and immune evasion, making it a key target in ASFV vaccine research.

In some embodiments, the full-length CD2V protein of the African swine fever virus has the amino acid sequence set forth in SEQ ID NO: 1.

To solve current challenges such as difficulties in purifying ASFV membrane proteins, low expression levels, and incorrect conformations in prokaryotic expression systems, the present disclosure analyzes the amino acid sequence of the CD2v protein. First, by analyzing the hydrophobicity distribution of the CD2v protein sequence, the extracellular region of the full-length CD2V protein is separated from its transmembrane and intracellular regions, obtaining the extracellular fragment sequence of the CD2V protein spanning positions 1 to 204. Subsequently, structural analysis of the CD2V protein reveals that its extracellular region primarily contains two relatively independent domains (domain 1 and domain 2).

While ensuring the integrity of these domains, the inventors expressed the two domains of the CD2v extracellular region separately and screened various truncated CD2v protein fragments, thereby obtaining multiple constructs of CD2v protein capable of eukaryotic expression. The results demonstrate that expressing the two extracellular domains of the CD2v protein as independent domains significantly improve the expression levels of both domains. Furthermore, when fragments from both domains of the CD2v protein are simultaneously constructed onto sixty-mer nanoparticles, a markedly enhanced immunogenic effect was observed, inducing high levels of anti-CD2V antibodies in immunized mice.

Unless otherwise defined, all technical or scientific terms used in the present disclosure have the same meaning as commonly used in the field to which the present disclosure belongs. For the purpose of interpreting this specification, the following definitions will apply and, where appropriate, terms used in the singular form shall also include the plural form and vice versa.

The expressions "a" and "an" as used herein include the plural unless the context clearly indicates otherwise. For example, reference to "a cell" includes a plurality of such cells and equivalents thereof as may be known to those skilled in the art.

The term "about" as used herein indicates a range of ±20% of the value following it. In some embodiments, the term "about" indicates a range of ±10% of the value following it. In some embodiments, the term "about" indicates a range of ±5% of the value following it.

The ASFV immunogenic composition described herein is preferably a subunit vaccine. The "subunit vaccine" described herein comprises one or more polypeptides or proteins derived from ASFV, or immunogenic fragments of the polypeptides or proteins, or one or more nucleic acid molecules encoding the immunogenic fragments of the polypeptides or proteins, and the nucleic acid molecules can be expressed in pigs. These polypeptides or proteins, immunogenic fragments of the polypeptides or proteins, or one or more nucleic acid molecules encoding the immunogenic fragments of the polypeptides or proteins can be prepared using techniques well known in the art.

The term "immunogenic composition" as used herein refers to a composition comprising at least one antigen that elicits an immunological response in a host or individual to which the immunogenic composition is administered. The immunological response may be a cellular and/or antibody-mediated immune response to the immunogenic composition of the present disclosure. Preferably, the immunogenic composition induces an immune response and more preferably confers protective immunity against one or more clinical signs of ASFV infection. Preferably, any host or individual referred to herein is an animal.

The range of values as used herein should be understood as having been enumerated for all numbers within that range. For example, the range of 1 to 20 should be understood to include any number, combination of numbers, or subrange from the following groups: 1, 2, 3, 4, 5, 6, 7, 8, 9, 10, 11, 12, 13, 14, 15, 16, 17, 18, 19, or 20.

The term "host cell" as used herein refers to eukaryotic cells, prokaryotic cells, or cells derived from multicellular organisms (e.g., cell lines) cultured as unicellular entities, either *in vivo* or *in vitro*, wherein the eukaryotic or prokaryotic cell may be, or has been, used as a recipient for nucleic acids, and includes the progeny of the original cell that has been genetically modified by the nucleic acid. It is understood that, due to natural, accidental, or intentional mutations, the progeny of a single cell may not necessarily be completely identical in morphology or in genomic or total DNA complement to the original parent. For example, the prokaryotic host cell of the present disclosure refers to a genetically modified prokaryotic host cell (e.g., a bacterium) produced by introducing a heterologous nucleic acid, such as an exogenous nucleic acid that is foreign (not naturally occurring) to the prokaryotic host cell or a recombinant nucleic acid not normally present in the prokaryotic host cell, into a suitable prokaryotic host cell; the eukaryotic host cell of the present disclosure refers to a genetically modified eukaryotic host cell produced by introducing a heterologous nucleic acid, such as an exogenous nucleic acid that is foreign to the eukaryotic host cell or a recombinant nucleic acid not normally present in the eukaryotic host cell, into a suitable eukaryotic host cell.

In some embodiments, the linker of the present disclosure may be a flexible peptide linker. In some embodiments, the peptide linker is rich in glycine, serine, alanine, proline and/or glutamine residues. In some embodiments, the peptide linker may be selected from (GₙS)ₘ, where n and m are each independently selected from integers from 0 to 5. For example, n is selected from 0, 1, 2, 3, 4, or 5, and m is selected from 1, 2, 3, 4, or 5. The term "linker" as used herein refers to a (peptide) linker of natural and/or synthetic origin, consisting of linear amino acids. The domains in the bispecific fusion polypeptide of the present disclosure may be linked via linkers, wherein each linker is fused and/or otherwise connected (e.g., via a peptide bond) to at least two polypeptides or domains. In some embodiments, the amino acid sequences of all linkers present in the bispecific fusion polypeptide of the present disclosure are identical. In other embodiments, the amino acid sequences of at least two linkers present in the bispecific fusion polypeptide of the present disclosure are different. The linker should have a length suitable for linking two or more monomeric domains in such a way that the linker is able to ensure that the different domains to which it is linked are correctly folded and appropriately presented so as to exert its biological activity function. In various embodiments, the linker has a flexible conformation. Suitable flexible linkers include, for example, those having glycine, glutamine and/or serine residues.

"Percentage (%) sequence identity" relative to a reference amino acid sequence refers to the percentage of amino acid residues in the candidate sequence that are identical to amino acid residues in the reference amino acid sequence after aligning the sequences and introducing gaps as needed to obtain the maximum percentage of sequence identity, without regard to any conservative substitutions as part of the sequence identity. To determine the percentage of amino acid sequence identity, alignments can be performed by various methods known in the art, such as using BLAST, ALIGN, or Megalign (DNASTAR) software. Those skilled in the art may determine appropriate parameters for aligning sequences, including any algorithms required to achieve optimal alignment over the full length of the sequences being compared.

Variants of the CD2V protein immunogenic fragment of the present disclosure may be obtained by substitution, addition or deletion of one or more amino acids, such that they have at least 85%, at least 90%, at least 95%, at least 96%, at least 97%, at least 98%, at least 99% or 100% amino acid sequence identity to the CD2V protein immunogenic fragment, while retaining comparable immunogenicity thereto.

The immunogenic variants of the CD2V protein immunogenic fragment of the present disclosure can be obtained by making one or more conservative amino acid substitutions to the CD2V protein immunogenic fragment. In some embodiments, conservative amino acid substitutions may refer to the replacement of one amino acid residue with a residue with biologically similar properties. Particularly preferred substitutions are generally conservative in nature, i.e., those that occur within amino acid families. For example, amino acids are generally divided into four families: (1) acidic - aspartic acid and glutamic acid; (2) basic - lysine, arginine, histidine; (3) non-polar - alanine, valine, leucine, isoleucine, proline, phenylalanine, methionine, tryptophan; and (4) uncharged polar - glycine, asparagine, glutamine, cysteine, serine, threonine, tyrosine. Phenylalanine, tryptophan and tyrosine are sometimes classified as aromatic amino acids. Examples of conservative substitutions include replacing one hydrophobic residue with another hydrophobic residue, such as isoleucine, valine, leucine, or methionine, or replacing one polar residue with another polar residue, such as arginine for lysine, glutamic acid for aspartic acid, or glutamine for asparagine; or making similar conservative substitutions of amino acids with structurally related amino acids, which substitutions do not have a significant impact on biological activity. Therefore, proteins having substantially the same amino acid sequence as the reference molecule but with a small number of amino acid substitutions that do not substantially affect protein immunogenicity are within the definition of the reference polypeptide.

The second domain used herein is capable of self-assembling into nanoparticles *in vitro* or pairing with another scaffold protein to assemble into nanoparticles. The immunogenic fragment of the CD2V protein or the variant thereof used herein forms a fusion protein with the second domain, and through the self-assembly or paired assembly of the second domain, the immunogenic fragment of the CD2V protein or the variant thereof is displayed on the surface of the nanoparticles.

In some embodiments, the second domain may be selected from some polypeptides synthesized *in vitro* that are capable of self-assembling into nanoparticles, such as LS, E2P, I3, etc. These polypeptides are capable of self-assembling into nanostructures in pairs *in vitro,* such as 60-mer nanostructures.

In one embodiment, the second domain is Lumazine synthase (LS). A monomeric LS subunit may be the full length LS protein, a single polypeptide or any part thereof, which is capable of directing the self-assembly of monomeric LS subunits into nanoparticles. Monomeric LS subunits from any known LS protein can be used to produce the recombinant protein of the present disclosure, as long as the monomeric LS subunit is capable of directing the self-assembly of the recombinant protein into nanoparticles displaying the CD2V protein immunogenic fragment on their surface. A representative LS protein has the amino acid sequence shown in SEQ ID NO: 17.

In one embodiment, the second domain is E2P. The monomeric E2P subunit may be the full-length E2P protein, a single polypeptide, or any part thereof, which is capable of directing the self-assembly of the monomeric E2P subunits into nanoparticles. Monomeric E2P subunits from any known E2P protein can be used to produce the recombinant protein of the present disclosure, as long as the monomeric E2P subunit can direct the self-assembly of the recombinant protein into nanoparticles displaying the CD2V protein immunogenic fragment on their surface. A representative E2P protein has the amino acid sequence set forth in SEQ ID NO: 18.

In one embodiment, the second domain is I3. The monomeric I3 subunit may be the full-length I3 protein, a single polypeptide, or any part thereof, which is capable of directing the self-assembly of the monomeric I3 subunits into nanoparticles. Monomeric I3 subunits from any known I3 protein can be used to produce the recombinant protein of the present disclosure, as long as the monomeric I3 subunit can direct the self-assembly of the recombinant protein into nanoparticles displaying the CD2V protein immunogenic fragment on their surface. A representative I3 protein has the amino acid sequence set forth in SEQ ID NO: 19.

In one embodiment, the second domain may be selected from some polypeptides synthesized *in vitro* that are capable of assembling into nanoparticles in pairs, such as I53-34A, I53-34B, I53-40A, I53-40B, I53-47A, I53-47B, I53-50A, I53-50B, I53-51A, I53-51B, I52-03A, I52-03B, I52-32A, I52-32B, I52-33A, I52-33B, I32-06A, I32-06B, I32-19A, I32-19B, I32-28A, I32-28B, I53-40A.1, I53-40B.1, I53-47A.1, I53-47A.1 NegT2, I53-47B.1, I53-47B.1 NegT2, I53-50A.1, I53-50A.1 NegT2, I53-50A.1 PostT1 and I53-50B4 PostT1. These polypeptides are capable of paired assembling into nanostructures *in vitro*, such as sixty-mer nanostructures.

The term "immunogenic composition" as used herein generally refers to a composition having a substance containing at least one antigen or an immunogenic portion thereof, which substance elicits a cellular immune response or an antibody-mediated immune response to the composition in a host. Preferably, the immunogenic composition induces an immune response, and more preferably, confers protective immunity against one or more of the clinical symptoms of ASFV infection. An immunogenic composition may also be referred to as a "vaccine" where the host exhibits a protective immune response such that resistance to new infection is enhanced and/or the clinical severity of the disease is reduced.

The term "pharmaceutically acceptable carrier" as used herein refers to a component in a pharmaceutical formulation, other than the active ingredient, that is non-toxic to a subject. Pharmaceutically acceptable carriers include, but are not limited to, buffers, excipients, stabilizers, or preservatives.

The term "prevention and/or treatment" as used herein refers to reducing the incidence of a specific ASFV infection, or reducing the severity of clinical symptoms caused by or associated with a specific ASFV infection. In addition, the term "prevention and/or treatment" may also refer to reducing the number of animals infected with a specific ASFV (i.e., reducing the incidence of ASFV infection) in a group of animals that have received an effective amount of the immunogenic composition as provided herein, compared to a group of animals that have not received the immunogenic composition as provided herein, or reducing the severity of clinical symptoms usually associated with or caused by ASFV infection.

The term "effective amount" as used herein refers to the amount that is effective in the necessary dosage and time period to achieve the desired therapeutic or prophylactic effect.

Examples and drawings are provided below to aid in understanding the present disclosure. It should be understood, however, that these Examples and drawings are intended to illustrate the present disclosure only, but do not constitute any limitation. The actual scope of protection of the present disclosure is set forth in the claims. It should be understood that any modifications and changes can be made without departing from the spirit of this disclosure.

To address technical problems such as the difficulty in purifying membrane proteins, low expression levels, and incorrect conformations in prokaryotic expression systems, as well as the problem that full-length CD2v is not expressed or is extremely difficult to express, the present disclosure analyzes the amino acid sequence of the structural protein CD2V of the African swine fever virus. First, by analyzing the hydrophobicity distribution of the protein sequence, the extracellular region of the full-length CD2V protein is separated from the transmembrane and intracellular regions to obtain the extracellular fragment sequence of the CD2V protein. Subsequently, structural analysis of the CD2V protein is performed to determine information such as disulfide bond positions and domains. While ensuring the integrity of these domains, various truncated CD2V protein fragments are screened, and multiple constructs of CD2V protein capable of being secreted and expressed in eukaryotic systems are obtained. These CD2V protein fragments induce high levels of anti-CD2V antibodies when administered to mice. The present disclosure decomposes the extracellular domain (positions 18-204) of CD2v into two independent domains for expression, and optimizes the best truncated constructs for each domain:
Domain 1: When the N-terminus is fixed at position 18 and the C-terminus is between positions 100 and 106, the expression level is significantly increased.
Domain 2: When the C-terminus is fixed at position 204 and the N-terminus is between positions 109 and 114, the expression level is significantly increased.

Furthermore, the inventors discovered that constructing the two domains of CD2v onto sixty-mer nanoparticles significantly enhances the immunogenicity, resulting in a substantial increase in antibody levels.

### Example 1: Construction of plasmid

The genes encoding different truncated forms of CD2v (domain 1: 10-100, 10-106, 10-109, 18-100, 18-106, 18-107, 18-109; domain 2: 108-204, 111-204, 114-204, 117-204) were linked to the genes encoding different multimeric scaffold proteins (I53-50A, I53-50B, I52-32A, I32-28B, LS, E2P, or I3) via a gene encoding a "linker" (SEQ ID NO: 21). The resulting recombinant eukaryotic gene fragments were obtained using overlap PCR, and a His-tag sequence was introduced at the 5' or 3' end of the gene to facilitate subsequent purification. The recombinant gene fragments were double-digested using the restriction endonucleases Sal I and EcoR I, and the digested fragments were ligated into the linearized pCMV-flag vector using DNA ligase to obtain eukaryotic recombinant plasmids. For the involved sequence information, refer to Table 1 below.

**Table 1**

| Protein Name | Description | SEQ ID NO |
|---|---|---|
| CD2v | Full length | SEQ ID NO: 1 |
| CD2v-10-100 | Truncation range 10-100 | SEQ ID NO: 2 |
| CD2v-10-106 | Truncation range 10-106 | SEQ ID NO: 3 |
| CD2v-10-109 | Truncation range 10-109 | SEQ ID NO: 4 |
| CD2v-18-100 | Truncation range 18-100 | SEQ ID NO: 5 |
| CD2v-18-106 | Truncation range 18-106 | SEQ ID NO: 6 |
| CD2v-18-107 | Truncation range 18-107 | SEQ ID NO: 7 |
| CD2v-18-109 | Truncation range 18-109 | SEQ ID NO: 8 |
| CD2v-108-204 | Truncation range 108-204 | SEQ ID NO: 9 |
| CD2v-111-204 | Truncation range 111-204 | SEQ ID NO: 10 |
| CD2v-114-204 | Truncation range 114-204 | SEQ ID NO: 11 |
| CD2v-117-204 | Truncation range 117-204 | SEQ ID NO: 12 |
| I53-50A | Scaffold protein | SEQ ID NO: 13 |
| I53-50B | Scaffold protein | SEQ ID NO: 14 |
| I52-32A | Scaffold protein | SEQ ID NO: 15 |
| I32-28B | Scaffold protein | SEQ ID NO: 16 |
| LS | Scaffold protein | SEQ ID NO: 17 |
| E2P | Scaffold protein | SEQ ID NO: 18 |
| I3 | Scaffold protein | SEQ ID NO: 19 |
| His | Tag protein | SEQ ID NO: 20 |
| Linker | / | SEQ ID NO: 21 |

### Example 2: Eukaryotic Expression of Recombinant Proteins

The eukaryotic expression of the recombinant protein CD2v comprises the following steps:
(1) Mass preparation of eukaryotic recombinant plasmids: 1 µg of each eukaryotic recombinant plasmid prepared in Example 1 was mixed with 100 µL of Top10 competent cells (purchased from Tsingke Biotechnology), and placed on ice for 15 minutes. After heat shock at 42°C for 90 seconds, the mixture was placed on ice for 5 minutes, followed by addition of antibiotic-free liquid LB medium, and cultured in a shaker at 37°C and 220 rpm for 40 minutes. After cultivation, the mixture was centrifuged at 2,000×g for 5 minutes, most of the supernatant was discarded, and the competent cells were resuspended in the remaining medium and evenly coated on solid LB (Amp+) plates, followed by incubation in a 37°C incubator for 12-16 hours. After incubation, single and well-grown colonies on the plates were picked for expanded culture, and plasmid extraction was performed according to the instructions of the Tiangen Plasmid Maxi Kit.
(2) Transfection of eukaryotic recombinant plasmids and cell culture: 1 mg of Eukaryotic recombinant plasmids were mixed with transfection reagent PEI (purchased from Polysciences), and the mixture was allowed to stand at room temperature for 5-10 minutes. 1 L of 293F cells at density of 3×10⁶ to 3.5×10⁶ cells/mL was prepared, and the prepared plasmid/PEI mixture was added dropwise to the cells with gentle shaking, followed by culture in an incubator at 37°C with 5% CO₂ at a rotation speed of 130 rpm for 4 days.
(3) Collection and concentration of culture supernatant: The cells cultured for 4 days as described in (2) were taken out, and the culture was centrifuged to harvest the supernatant, which was filtered through a 0.45 µm filter membrane to remove cell debris. The filtered supernatant was subjected to ultrafiltration and concentration by membrane cassette method (15 kD), and the concentrated solution was diluted with buffer (1×PBS buffer, pH 8.0) for later use.
(4) Protein purification: A Ni-affinity chromatography column was equilibrated with equilibration buffer (1×PBS buffer, pH 8.0), and the supernatant was loaded onto the Ni-affinity chromatography column. The column was rinsed with 5-10 column volumes of washing buffer (1×PBS buffer, 20 mM imidazole, pH 8.0), and re-equilibrated with equilibration buffer until baseline stabilization. The target protein was eluted with elution buffer (1×PBS buffer, 500 mM imidazole, pH 8.0). The eluted protein solution was sterilized by filtration through a 0.22 µm filter membrane, and the protein concentration was determined by NanoDrop. The expression levels of each construct are shown in Tables 2 and 3.

**Table 2. Expression levels of extracellular domain 1 of the African swine fever CD2v protein (Unit: mg/L)**

| | 10-100 | 10-106 | 10-109 | 18-100 | 18-106 | 18-107 | 18-109 |
|---|---|---|---|---|---|---|---|
| I53-50A-his | <0.1 | <0.1 | <0.1 | >4 | >3 | <0.1 | <0.1 |
| I53-50B-his | <0.1 | / | / | >8 | >5 | <0.5 | <0.1 |
| I52-32A-his | <0.1 | <0.1 | <0.1 | >4 | >2 | <0.5 | <0.5 |
| I32-28B-his | <0.1 | / | / | >5 | >4 | <0.5 | <0.5 |
| LS-his | <0.1 | <0.1 | <0.1 | <0.5 | <0.5 | <0.1 | <0.1 |
| E2P-his | <0.1 | / | / | >5 | >5 | <0.5 | <0.5 |
| I3-his | <0.1 | / | / | >2 | >2 | <0.5 | <0.5 |
| His | <0.1 | <0.1 | <0.1 | <0.5 | <0.5 | <0.5 | <0.5 |

**Table 3. Expression levels of extracellular domain 2 of the African swine fever CD2v protein (Unit: mg/L)**

| | 108-204 | 111-204 | 114-204 | 117-204 |
|---|---|---|---|---|
| I53-50A-his | >5 | >5 | >8 | <0.5 |
| I53-50B-his | >4 | >6 | >12 | <0.5 |
| I52-32A-his | >5 | >8 | >10 | <0.5 |
| I32-28B-his | / | >8 | >12 | <0.5 |
| LS-his | / | >3 | >6 | <0.5 |
| E2P-his | / | >3 | >8 | <0.5 |
| I3-his | / | >5 | >8 | <0.5 |
| His | <0.5 | <0.5 | <0.5 | <0.5 |

### Example 3: 60-mer Assembly Experiments of each of Purified Antigens

(1) Assembly of two-component nanoparticles: Purified recombinant proteins were mixed separately with the corresponding paired scaffold proteins; Negative-stained grids were prepared for samples, and the assembly effect of nanoparticles was examined under a 120 kV electron microscope. The assembly results of the recombinant proteins, exemplified by the 18-100 truncation of domain 1 and the 114-204 truncation of domain 2, are summarized in Tables 4 and 5 below.
(2) Assembly of single-component nanoparticles: Single-component nanoparticles assembled spontaneously without a separate assembly step. Negative-stained grids were prepared for samples, and the assembly effect of nanoparticles was examined under a 120 kV electron microscope. The assembly results of the recombinant proteins, exemplified by the 18-100 truncation of domain 1 and the 114-204 truncation of domain 2, are summarized in Tables 4 and 5 below.

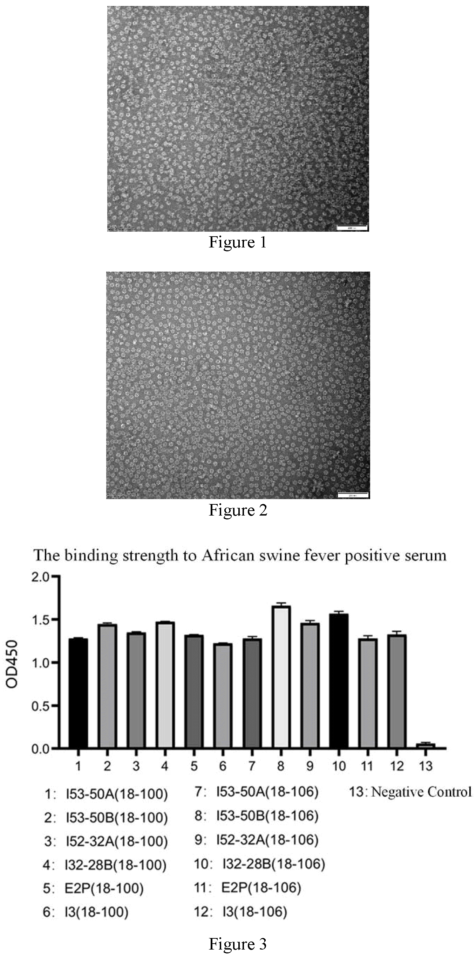

The electron microscopy results of the exemplary assembly of the 18-100 truncation of domain 1 fused with the I3 protein are shown in Figure 1.

The electron microscopy results of the exemplary assembly of the 114-204 truncation of domain 2 fused with I53-50A and I53-50B are shown in Figure 2.

**Table 4. Statistics on the sixty-mer assembly effect of extracellular domain 1 of the African swine fever CD2v protein**

| 18-100 Truncation | Polymerization State | 60-mer Particle Assembly Mode | Assembly Effect |
|---|---|---|---|
| I53-50A | Trimer | Assembly with I53-50B | + |
| I53-50B | Pentamer | Assembly with I53-50A | + |
| I52-32A | Dimer | Assembly with I52-32B | + |
| I32-28B | Pentamer | Assembly with I32-28A | + |
| E2P | 60-mer | Self-Assembly | + |
| I3 | 60-mer | Self-Assembly | + |

| | | | |
|---|---|---|---|
| Note: In the assembly effect, + indicates that 60-mer particles can be formed by assembly, and - indicates that 60-mer particles cannot be formed by assembly. | | | |

**Table 5. Statistics on the sixty-mer assembly effect of extracellular domain 2 of the African swine fever CD2v protein**

| 114-204 Truncation | Polymerization State | 60-mer Particle Assembly Mode | Assembly Effect |
|---|---|---|---|
| I53-50A | Trimer | Assembly with I53-50B | + |
| I53-50B | Pentamer | Assembly with I53-50A | + |
| I52-32A | Dimer | Assembly with I52-32B | + |
| I32-28B | Pentamer | Assembly with I32-28A | + |
| E2P | 60-mer | Self-Assembly | + |
| I3 | 60-mer | Self-Assembly | + |

| | | | |
|---|---|---|---|
| Note: In the assembly effect, + indicates that 60-mer particles can be formed by assembly, and - indicates that 60-mer particles cannot be formed by assembly. | | | |

### Example 4: African Swine Fever Positive Serum Binding Assay

Each of the purified recombinant proteins obtained in Example 2 or the assembled particles obtained in Example 3 were diluted to 1 µg/mL with 1×PBS (pH 8.0) as coating working solution, and 100 µL of the diluted proteins was added to each well of a 96-well microplate, followed by incubation at 4 °C overnight (12-16 hours). The microplate was taken out, the liquid in the wells was discarded, and the plate was washed three times with PBST (1×PBS containing 0.5‰ Tween 20) and patted dry on absorbent paper. 200 µL of blocking buffer (PBST containing 0.2% BSA) was added to each well, and the plate was left at room temperature for 1 hour. The blocking solution was discarded, and the plate was washed three times with PBST and patted dry. African swine fever positive serum (purchased from China Institute of Veterinary Drug Control) was diluted 1:1000 with blocking solution, and 100 µL of the diluted serum was added to the antigen-coated microplate, followed by incubation at room temperature for 60 minutes. The plate was washed three times with PBST, then 100 µL of goat anti-swine IgG-HRP marker diluted 1:5000 with blocking solution was added to each well, and incubation was performed at room temperature for 60 minutes. The plate was washed five times with PBST and patted dry. 100 µL of TMB substrate was added to each well, and the reaction was carried out at room temperature in the dark for 2-10 minutes, followed by addition of 50 µL stop solution (2 M sulfuric acid) to each well. The absorbance at 450 nm was measured with 630 nm as the reference wavelength. A sample was judged positive when the absorbance value was more than 2.0-fold that of the negative control (cell culture medium).

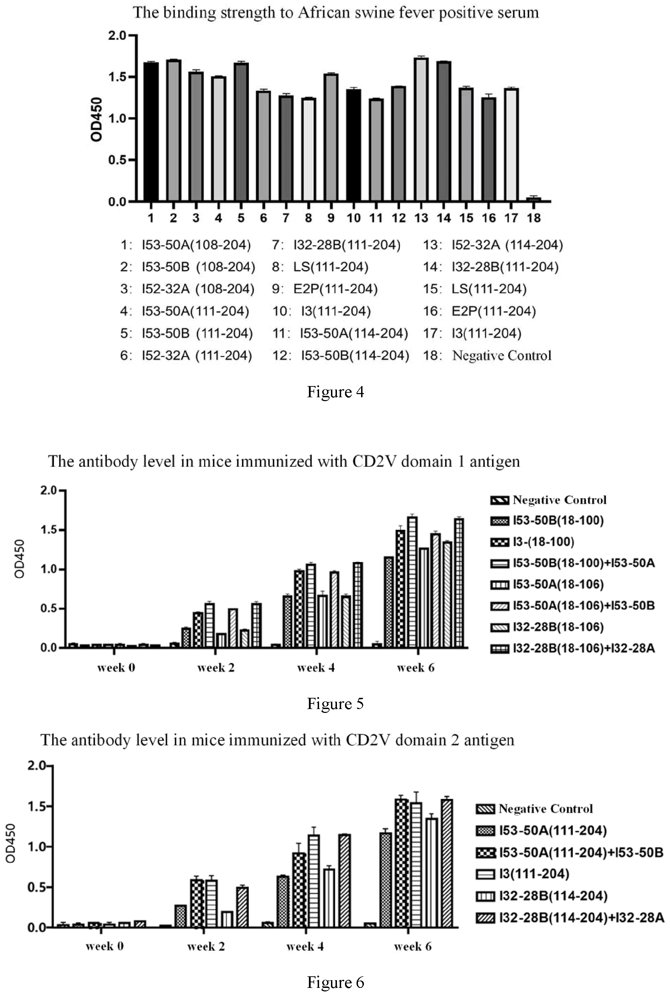

The results of the exemplary truncated recombinant proteins of domain 1 and domain 2, or their assembled particles, are shown in Figure 3 and Figure 4, respectively. The results indicate that each of the purified recombinant proteins of CD2v obtained in Example 2 or their assembled particles can be bound by African swine fever positive serum, demonstrating that the above-mentioned recombinant proteins or their assembled particles expressed in the present disclosure possess the correct spatial conformation.

### Example 5: Antigen Immunogenicity Assay

(1) Six-week-old BALB/c mice were immunized with the partially purified CD2v recombinant protein antigens from Example 2 and the CD2v sixty-mer antigens assembled in Example 3 by conventional methods. For the primary immunization, 10 µg of antigen was subcutaneously injected with Freund's complete adjuvant; A secondary immunization was performed 4 weeks later at 10 µg per mouse with Freund's incomplete adjuvant.
(2) Blood samples were collected before primary immunization (week 0), 2 weeks after primary immunization (week 2), 4 weeks after primary immunization (week 4), and 2 weeks after secondary immunization (week 6). Subsequently, antibody levels were assayed by ELISA.
(3) Purified CD2V antigen was diluted to 1 µg/mL with 1×PBS (pH 8.0) as coating working solution, and 100 µL of the diluted protein was added to each well of a 96-well microplate, followed by incubation at 4 °C overnight (12-16 hours). The microplate was taken out, the liquid in the wells was discarded, and the plate was washed three times with PBST (1×PBS containing 0.5‰ Tween 20) and patted dry on absorbent paper. 200 µL of blocking buffer (PBST containing 0.2% BSA) was added to each well, and the plate was left at room temperature for 1 hour. The blocking solution was discarded, and the plate was washed three times with PBST and patted dry. Sera from immunized mice at each time point were diluted 1:1000 with blocking solution, and 100 µL of the diluted serum was added to each antigen-coated well, followed by incubation at room temperature for 60 minutes. The plate was washed three times with PBST. Then, goat anti-mouse IgG-HRP marked antibody diluted 1:10000 with blocking solution was added to each well, followed by incubation at room temperature for 60 minutes. The plate was washed five times with PBST and patted dry. 100 µL of TMB substrate was added to each well, and the reaction was carried out at room temperature in the dark for 2-10 minutes, followed by addition of 50 µL stop solution (2 M sulfuric acid) to each well. The absorbance at 450 nm was measured with 630 nm as the reference wavelength. A sample was judged positive when the absorbance value was more than 2.0-fold that of the negative control (cell culture medium).

The serum antibody levels in mice immunized with exemplary recombinant proteins of the 18-100 truncation and the 18-106 truncation of domain 1, as well as the recombinant protein of the 114-204 truncation of domain 2, or their assembled particles, are shown in Figure 5 and Figure 6, respectively. The results demonstrate that compared to monomeric antigens with heterogeneous aggregation states, the CD2V antigen assembled into sixty-mer nanoparticles exhibits significantly higher immunogenicity.

The technical solutions of the present disclosure are not limited to the limitations of the above specific examples, and all technical variants made according to the technical solutions of the present disclosure fall within the scope of protection of the present disclosure.

## Claims

1. An immunogenic fragment or an immunogenic variant thereof, **characterized in that** the immunogenic fragment comprises at least an amino acid fragment at positions 18 to 100 of the amino acid sequence shown in SEQ ID NO: 1, and/or comprises at least the amino acid fragment at positions 117 to 200 of the amino acid sequence shown in SEQ ID NO: 1.

2. The immunogenic fragment or the immunogenic variant thereof according to claim 1, **characterized in that** the immunogenic fragment comprises at least the amino acid fragment at positions 18 to 106 of the amino acid sequence shown in SEQ ID NO: 1; and/or
the immunogenic fragment comprises at least the amino acids at positions 114 to 204 of the amino acid sequence shown in SEQ ID NO: 1.

3. The immunogenic fragment or the immunogenic variant thereof according to claim 1, **characterized in that** the immunogenic fragment has the amino acid sequence shown in SEQ ID NO: 5, SEQ ID NO: 6, SEQ ID NO: 7, or SEQ ID NO: 8, or an amino acid sequence having at least 85% sequence identity thereto; and/or has the amino acid sequence shown in SEQ ID NO: 9, SEQ ID NO: 10, or SEQ ID NO: 11, or an amino acid sequence having at least 85% sequence identity thereto.

4. A recombinant protein, **characterized in that** the recombinant protein comprises:
a first domain comprising the immunogenic fragment or the immunogenic variant thereof according to any one of claims 1 to 3; and,
a second domain comprising a scaffold polypeptide for forming nanoparticles.

5. The recombinant protein according to claim 4, **characterized in that** the second domain is selected from at least one of I53-34A, I53-34B, I53-40A, I53-40B, I53-47A, I53-47B, I53-50A, I53-50B, I53-51A, I53-51B, I52-03A, I52-03B, I52-32A, I52-32B, I52-33A, I52-33B, I32-06A, I32-06B, I32-19A, I32-19B, I32-28A, I32-28B, I53-40A.1, I53-40B.1, I53-47A.1, I53-47A.1 NegT2, I53-47B.1, I53-47B.1 NegT2, I53-50A.1, I53-50A.1 NegT2, I53-50A.1 PostT1, I53-50B4 PostT1, LS, E2P and I3,
preferably, the first domain and the second domain are connected either directly or via a linker.

6. A nucleic acid molecule, **characterized in that** the nucleic acid molecule encodes the immunogenic fragment or the immunogenic variant thereof according to any one of claims 1 to 3, or encodes the recombinant protein according to claim 4 or 5.

7. A host cell, **characterized in that** the host cell comprises the nucleic acid molecule according to claim 6, or is capable of expressing the immunogenic fragment or the immunogenic variant thereof according to any one of claims 1 to 3 or the recombinant protein according to claim 4 or 5;
preferably, the host cell is a prokaryotic cell or a eukaryotic cell.

8. A nanoparticle, **characterized in that** the nanoparticle comprises the immunogenic fragment or the immunogenic variant thereof according to any one of claims 1 to 3 or the recombinant protein according to claim 4 or 5,
preferably, the immunogenic fragment or the variant thereof is displayed on the surface of the nanoparticle.

9. An immunogenic composition, **characterized in that** the immunogenic composition comprises: the immunogenic fragment or the immunogenic variant thereof according to any one of claims 1 to 3, the recombinant protein according to claim 4 or 5, the nucleic acid molecule according to claim 6, the host cell according to claim 7, or the nanoparticle according to claim 8; and a pharmaceutically acceptable carrier,
preferably, the immunogenic composition further comprises an additional African swine fever virus antigen.

10. Use of the immunogenic fragment or the immunogenic variant thereof according to any one of claims 1 to 3, the recombinant protein according to claim 4 or 5, the nucleic acid molecule according to claim 6, the host cell according to claim 7, the nanoparticle according to claim 8, or the immunogenic composition according to claim 9 in the preparation of a medicament for preventing and/or treating African swine fever virus infection in a subject.

11. The use according to claim 10, **characterized in that**, the subject includes a mammal,
preferably, the subject comprises Suidae animal or pig,
more preferably, the subject comprises a wild boar (*Sus scrofa*), domestic pig (*Sus scrofa domesticus*), warthog (*Potamochoerus*), forest hog (*Hylochoerus*), giant forest hog (*Hylochoerus*), African wild boar (*Potamochoerus*) and feral pig.

12. The use according to claim 10 or 11, **characterized in that**, the African swine fever virus infection is a pathogenic African swine fever virus,
preferably, the disease or symptom of the African swine fever virus infection is selected from the group consisting of: African swine fever, acute African swine fever, chronic African swine fever, death from illness, death, sudden death, fever, high fever, anorexia, lethargy, weakness, lack of appetite, recumbency, erythema, cyanotic skin maculopathy, dysentery, constipation, abdominal pain, respiratory symptoms, cough, vomiting, dyspnea, nasal discharge and conjunctival secretions, hemorrhage, epistaxis, abortion, leukopenia, and thrombocytopenia.

13. A kit for detecting African swine fever virus infection, **characterized in that** the kit comprises the immunogenic fragment or the immunogenic variant thereof according to any one of claims 1 to 3 or the recombinant protein according to claim 4 or 5.

14. Use of the immunogenic fragment or the immunogenic variant thereof according to any one of claims 1 to 3 or the recombinant protein according to claim 4 or 5 in the preparation of a kit for detecting African swine fever virus infection,
preferably, the African swine fever virus is from a body fluid or tissue sample in a subject,
more preferably, the body fluid sample is selected from a blood, saliva or serum sample.
